# EUROPEAN PATENT SPECIFICATION

(11) **EP 2 752 195 B1**
(45) Date of publication and mention of the grant of the patent: **16.01.2019**
(21) Application number: 11870524.3
(22) Date of filing: 10.08.2011
(51) Int. Cl.: A61K 36/87, A61P 3/06, A61K 45/06, A61K 31/352, A61K 31/353, A61K 31/366, A23L 33/105

(54) **GRAPE EXTRACT, NUTRITIONAL SUPPLEMENT COMPRISING GRAPE EXTRACT, AND THE USE THEREOF AS A FUNCTIONAL INGREDIENT**
WEINTRAUBENEXTRAKT, NAHRUNGSERGÄNZUNGSPRÄPARAT MIT DEM WEINTRAUBENEXTRAKT UND VERWENDUNG DAVON ALS FUNKTIONELLER BESTANDTEIL
EXTRAIT DE RAISIN, COMPLÉMENT NUTRITIONNEL LE COMPRENANT ET SON UTILISATION COMME INGRÉDIENT FONCTIONNEL

(43) Date of publication of application: 09.07.2014
(73) Proprietor: Abro Biotec, S.L., 47359 Valbuena de Duero (Valladolid) (ES)
(72) Inventor: GUADARRAMA RODRÍGUEZ, Alberto, E-47359 Valdebuena de Duero (Valladolid) (ES); VILLANUEVA SÁNCHEZ, Sonia, E-47359 Valdebuena de Duero (Valladolid) (ES); SANZ BUENHOMBRE, Marisa, E-47359 Valdebuena de Duero (Valladolid) (ES); YUBERO POSTILLO, Noemí, E-47359 Valdebuena de Duero (Valladolid) (ES); GARRIDO LAFUENTE, Ignacio, E-47359 Valdebuena de Duero (Valladolid) (ES); PINTO SOLANO, Julio Andrés, E-47359 Valdebuena de Duero (Valladolid) (ES)
(74) Representative: Lehmann Novo, Maria Isabel
(86) International application number: PCT/ES2011/070593
(87) International publication number: WO 2013/021076

(56) References cited:
- WO-A1-97/39632
- ES-A1- 2 217 966
- ES-A1- 2 319 032
- US-A1- 2004 166 179
- US-A1- 2010 015 316
- US-A1- 2011 177 182
- US-B1- 6 238 673
- WANG X ET AL: "Chemical characterization and antioxidant evaluation of muscadine grape pomace extract", FOOD CHEMISTRY, ELSEVIER LTD, NL, vol. 123, no. 4, 15 December 2010 (2010-12-15), pages 1156-1162, XP027155715, ISSN: 0308-8146 [retrieved on 2010-05-24]
- EDUARDO PASTRANA-BONILLA ET AL: "Phenolic Content and Antioxidant Capacity of Muscadine Grapes", JOURNAL OF AGRICULTURAL AND FOOD CHEMISTRY, vol. 51, no. 18, 1 August 2003 (2003-08-01), pages 5497-5503, XP055179728, ISSN: 0021-8561, DOI: 10.1021/jf030113c
- SANDHU A K ET AL: "Effects of exogenous abscisic acid on antioxidant capacities, anthocyanins, and flavonol contents of muscadine grape (Vitis rotundifolia) skins", FOOD CHEMISTRY, ELSEVIER LTD, NL, vol. 126, no. 3, 1 June 2011 (2011-06-01), pages 982-988, XP027600997, ISSN: 0308-8146, DOI: 10.1016/J.FOODCHEM.2010.11.105 [retrieved on 2011-01-13]
- RODRIGO, R. ET AL.: 'Modulation of endogenous antioxidant system by wine polyphenols in human disease.' CLINICA CHIMICA ACTA vol. 412, no. 5-6, 20 February 2011, pages 410 - 424, XP027587723
- RODRIGUEZ MONTALEGRE, R. ET AL.: 'Phenolic compounds in skins and seeds of ten grape Vitis vinifera varieties grown in a warm climate.' JOURNAL OF FOOD COMPOSITION AND ANALYSIS vol. 19, no. 6-7, pages 687 - 693, XP024929655

## Description

### TECHNICAL FIELD

The present invention falls within the food and/or pharmaceutical sector, and in particular, it relates to functional ingredients made up of a grape-derived polyphenol extract with high antioxidant potential.

### STATE OF THE ART PREVIOUS TO THE INVENTION

In nature, oxidation refers to any process in which loss of electrons, oxygen uptake, or transfer of hydrogen (dehydrogenation) takes place. By contrast, reduction is the process whereby electrons are taken up or oxygen atoms are lost. An oxidation process is always accompanied by a reduction process (Elejalde JI, 2001).

Free oxygen radicals or reactive oxygen species (ROS) refer to molecules whose atomic structure has an unpaired electron in its outer orbit. Said configuration makes them highly unstable and extremely reactive. They are characterized by an exceedingly short lifespan and a high capacity to combine non-specifically with any molecule in the cell structure, such as carbohydrates, lipids, proteins, nucleic acids and derivatives of each of the above. The most important of the inorganic ROS are molecular oxygen (O₂), the anion superoxide (O₂⁻) and the hydroxyl radical (OH⁻), as well as its immediate precursor, hydrogen peroxide (H₂O₂). Of the secondary and organic ROS, it is worth mentioning the radical peroxyl (ROO⁻), organic hydroperoxide (ROOH) and the peroxidized lipids (Elejalde JI, 2001).

ROS are continuously produced in organisms as products of the cells' normal metabolism. They play an essential role in certain biological processes, such as the enzymatic reactions of the mitochondrial respiratory chain (Chance B et al., 1979), the detoxification reactions carried out by cytochrome P-450, phagocytosis (Klebanoff SJ, 1980), or the synthesis of prostaglandins (Porter NN, 1986). However, these radicals, which are indispensable for cells to function properly, may also cause cellular damage when their production is unchecked.

### Biological sources of free radicals

Mitochondria are the body's main source of ROS. These molecules are generated mainly at the level of the electron transport chain. As they pass through the inner mitochondrial membrane, they generate an electron gradient that supplies the energy necessary to form ATP (adenosine triphosphate). In this process of oxidative phosphorylation, oxygen acts as the final receptor of the electrons. Between the initial nutrients and the final generation of energy, various molecules with different degrees of oxidation are formed, some of which can shed 1 or 2 electrons to oxygen and produce partially reduced intermediaries or ROS (Turrens J, 1994).

Other significant sources of ROS are peroxisomes, cytosolic organelles that are very rich in oxidases that generate high quantities of H₂O₂; polymorphonuclear leukocytes, which through the enzyme NADPH oxidase generate O₂, which in turn transforms into O₂⁻ in the presence of iron (during inflammation); or the enzyme xanthine dehydrogenase, present in the endothelia, and whose function is to purify the xanthines, thus generating O₂⁻.

### Toxicity of free radicals

The mid-fifties saw the publication of the first study to highlight the role of free radicals as toxic, disease-generating agents (Gerschman R, 1954).

In the case where the molecule is a lipid, damage is caused to the structures where it is present, such as cell membranes or lipoproteins. In the case of membranes, this process alters their permeability, leading to cell death. In the case of lipoproteins, it is known that the oxidation of LDLs (low-density lipoproteins) plays a crucial role in the formation of atheromatous plaque (Hazel SL, 2000). The process whereby lipids become oxidized, known as lipid peroxidation, generates various by-products such as MDA (malonyldialdehyde), the detection of which in tissues, plasma or urine is one of the most standard methods of assessing oxidative stress.

When the oxidized molecule is a protein, the oxidation of the aminoacids leads to cross-linking of the peptide chains, protein fragmentation, and loss of functionality.

Lastly, in the case of DNA, the oxidation of nucleic acids produces modified bases, which give rise to mutations that can become involved in loss of gene expression or in processes of carcinogenesis.

### Diseases, degenerative processes and oxidative stress

Oxidative stress is defined as an alteration of the pro-oxidant/antioxidant balance in favor of the former (Sies H, 1986). ROS have been shown to play an important role in various pathological processes, a selection of which is described below:
- Ageing: the theory of ROS and ageing is based on the idea that ageing results from the accumulation of organic lesions due to the action of these molecules (Harman D, 1993) . It has also been found that there is less proteolytic activity in aged cells, a decrease in antioxidant concentration, and inactivation of the detoxifying enzymes responsible for the elimination of ROS. Furthermore, an accumulation of non-degraded oxidized proteins has been observed.
- Atherosclerosis: it is known that the formation of atheromatous plaque begins with the uptake of low-density lipoproteins (LDL) by macrophages. The latter accumulate in the subendothelial space, where they induce the migration, proliferation and hypertrophy of muscle cells. Under certain oxidative conditions, LDLs become oxidized, giving them or their by-products a greater atherogenic potential, as they are more easily taken up by the macrophages. In this way, they stimulate the production of adhesion factors, thrombotic factors and proliferation factors, thus creating the atherogenic lesion or causing it to spread (Hazen SL, 2000). It has been shown that there is a close relationship between ROS and the oxidation of LDL, and it is known that an increase thereof is indicative of the onset of atherosclerosis (Elejalde JI, 2001).
- Cancer: the transformation of normal cells into carcinogenic cells is conditioned by the presence of mutations in genes or oncogenes that control key cell functions, which may take place in a cellular redox state. Decreased antioxidant enzyme levels have been detected in various types of tumor cells (Oberley TD & Oberley LW, 1997). It has also been suggested that oxidative stress plays a role in the progression of angiogenesis. Moreover, the activation of certain early genes that could take part in controlling the transcription of growth factors necessary for tumor development has also been observed.
- Other pathological processes in which the involvement of ROS has been described are acute and chronic kidney failure, diabetes mellitus, high blood pressure, cirrhosis, liver failure, etc. (Elejalde JI, 2001).

### Polyphenols

Polyphenols are chemical compounds or substances found in a large number of plant-derived foods. The term comprises a wide variety of molecules with a common structure, characterized by having several hydroxyl groups attached to aromatic rings. However, polyphenols also include molecules with a phenol ring, as in the case of phenolic acids or phenolic alcohols (D'Archivio M et al., 2007) .

### Classification of polyphenols

Polyphenols are classified based on the number of phenol rings they contain, as well as the structural elements that join these rings together. As such, the main polyphenol groups are:
- Flavonoids: which have a carbon skeleton of diphenylpropane and two benzene rings joined together by a linear chain of 3 carbon atoms. To date, more than 6,000 flavonoids have been identified in plants, and the list continues to grow. Flavonoids can be divided into 6 subclasses, based on the state of oxidation of the central pyran ring (D'Archivio M et al., 2007):
   - Flavonols: which have a double bond between carbons 2 and 3, with a hydroxyl group at C₃. They are the most ubiquitous flavonoids in foods, and quercetin is the most representative. The principal sources of flavonols are onions, kale, leeks, broccoli and blackberries. Tea and red wine contain approximately 45 and 30 mg of flavonols/L, respectively. It is important to point out that the synthesis of flavonols is stimulated by light, which means that they accumulate in the most external parts of fruit.
   - Flavones: which have a double bond between carbons 2 and 3, and are the least common flavonoids. They are found in parsley and in celery.
   - Flavanones: which are characterized by having a saturated chain of 3 carbon atoms and one oxygen atom at carbon 4. They are only found at high concentrations in citrus fruit, although they are also present in tomatoes and in some aromatic plants such as mint.
   - Isoflavones: which are structurally similar to estrogens, and in fact can bind to their receptors, which is why they are also known as phytoestrogens. Soy and its derivatives are the principal sources of isoflavones.
   - Anthocyanins: which are the pigments responsible for the red, blue and purple coloring of fruit, flowers and other plant tissues and products. They are found in various dietary products, such as red wine, some varieties of grain, and some vegetables like onions, radishes, cabbage or beans.
   - Flavanols: which have a saturated chain of 3 carbons, with a hydroxyl group at C₃. They exist both as monomers and as polymers, known as catechins and proanthocyanidins, respectively. The principal flavanols in fruit are catechin and epicatechin, whereas gallocatechin, epigallocatechin and epigallocatechin gallate are essentially found in tea. Catechins are found in fruits such as apricots and cherries and in other products such as tea, chocolate and white wine.
- Phenolic acids: which are divided into two groups, derivatives of benzoic acid and derivatives of cinnamic acid:
   - Hydrobenzoic acids: which are found in very few plants consumed by humans, and so are not considered to be of particular interest for nutrition. Among them, gallic acid is worth mentioning.
   - Hydroxycinnamic acids: which are mainly represented by coumaric acid, caffeic acid and ferulic acid. They are found in all parts of fruit, though they are most heavily concentrated in the outermost part of ripe fruit.
- Phenolic alcohols: tyrosol and hydroxytyrosol are the principal types. They are mainly present in olive oil. Tyrosol is also found in wine, both white and red, and in beer; hydroxytyrosol, on the other hand, is found in red wine.
- Stilbenes: the principal stilbene present in the diet of humans is resveratrol, although the amount of these compounds ingested in the diet is small. Stilbenes are produced by plants as a response to pathogens or to certain stress conditions. They have been detected in more than 70 plant species, such as grapes, berries and peanuts.
- Lignans: lignans are produced by the oxidative dimerization of two phenylpropane units. The principal source of these compounds is flax seed. Interest in the study of lignans has been on the rise in recent years because of their potential applications in anticancer chemotherapy and other pharmacological effects.

### Polyphenols: prevention and treatment of diseases

Various epidemiological studies have suggested that the consumption of fruits and vegetables has a protective effect against diseases such as cancer or cardiovascular pathologies. Many authors attribute this effect to the fact that they contain polyphenols, which, as mentioned above, are compounds with a high antioxidant potential that are most characteristically found in fruits and vegetables. In addition to their antioxidant properties, polyphenols have proven to engage in other activities that could be highly relevant in preventing and treating a variety of pathologies. For example, they are able to chelate ROS, regulate the production of nitric oxide, decrease the immobilization of leukocytes, induce apoptosis mechanisms, inhibit cell proliferation and angiogenesis, and have even been observed to engage in phytoestrogen activities (Higdon JV & Frei B, 2003).

### Cardiovascular disease

More and more evidence points to the existence of a direct correlation between the consumption of foods and drinks rich in polyphenols and the incidence of cardiovascular disease in the population. Among these foods and drinks, we find fruit, vegetables, cacao and red wine.

The concept known as "the French paradox" is a term coined by several epidemiologists highlighting the relatively low rate of coronary disease among the population of France, despite being a population with high levels of saturated fat consumption, a factor closely linked to an increase in cardiovascular disease.

More extensive epidemiological studies, such as the MONICA project, have highlighted the declining slope in the rate of coronary events from northern to southern Europe (Kuulasmaa K et al., 2000). Interestingly, this relatively low range of coronary events found in France and other southern European countries was associated with cardiovascular risk factors comparable to those found in developed countries with a much greater rate of cardiovascular events (Stoclet JC et al., 2004). With this information in mind, the polyphenol content of wine has received wide attention for two reasons: on the one hand, because it is particularly high (more than 0.2% in red wine), and on the other hand because of the possible involvement of polyphenols and "the French paradox", given France's high levels of wine consumption (Stoclet JC et al., 2004).

It is believed that some of the chemical properties and biological effects of polyphenols could be involved in protecting against cardiovascular disease through a variety of mechanisms:
First of all, these molecules' antioxidant properties could protect blood vessels against damage caused by the oxidative stress associated with the majority of cardiovascular risk factors. As such, it has been suggested that flavonoids could protect endothelial antithrombotic factors, such as NO or prostacyclin, from breakdown owing to their superoxide scavenging effect. In fact, the ability of the procyanidin oligomers of condensed tannins to chelate peroxynitrate protects the endothelial cells from the oxidation of the lipids in their membrane and from cytotoxicity (Aldini G et al., 2003) .

It has moreover been proposed that the antioxidant properties of polyphenols could protect the functioning of the vascular endothelium from the effects of the oxidation of LDLs, which affect the capacity of the endothelium to induce relaxation.

Some studies have highlighted that consuming wine, polyphenols derived from red wine, or black grape juice boosts plasma's antioxidant capacity and reduces the oxidation of circulating LDLs (Nigdikar SV et al., 1998). It has also been determined that following consumption of black grape juice, these effects were associated with the restoration of endothelial function in patients with coronary disease.

In addition to slowing LDL oxidation, some polyphenols such as caffeic acid can act as cytoprotective agents. In this way, their actions would inhibit apoptosis in endothelial cells by blocking the signaling pathways activated by oxidized LDL (Vieira O et al., 1998).

It has also been highlighted that polyphenols are capable of increasing the generation of NO by the enzyme endothelial NO synthase (eNOS) (Aldini G et al., 2003). In all of the studies, plant-derived polyphenols induced endothelium-dependent vascular relaxation that was usually associated with an increase in the formation of cyclic GMP (cGMP) and which was not produced in the presence of NOS inhibitors, which indicated that this response was mediated by the cCMP-NOS-pathway. Moreover, the effect of relaxation was directly proportional to the concentration of polyphenols in the wine (Burns J et al., 2000).

Other noteworthy effects of polyphenols, as far as protection of the cardiovascular system is concerned, are increased eNOS expression; relaxation and hyperpolarization of the endothelium mediated by EDHF; increase in the release of prostacyclin by the endothelial cells; inhibition of endothelin-1 synthesis; as well as vasodilator and antihypertensive effects.

Document WANG X ET AL, "Chemical characterization and antioxidant evaluation of muscadine grape pomace extract", FOOD CHEMISTRY, ELSEVIER LTD, NL, vol. 123, no. 4, ISSN 0308-8146, discloses the provision of a muscadine grape pomace extract having a high content of polyphenols, which has antioxidative effects. In particular, in table 2 a muscadine pomace extract is disclosed comprising 0,62 mg/g of ellagic acid, 0,17 mg/g of myricetin, 0,2 mg/g of quercetin, 1,46 mg/g of catechin, 1,28 mg/g of epicatechin and 0,9 mg/g of epigallocatechin. The total phenolics content of the extract is 34,1 mg/g of gallic acid equivalents and 3 mg/g of quercetin equivalents.

Document EDUARDO PASTRANA-BONILLA ET AL, "Phenolic Content and Antioxidant Capacity of Muscadine Grapes", JOURNAL OF AGRICULTURAL AND FOOD CHEMISTRY, vol. 51, no. 18, ISSN 0021-8561, pages 5497 - 5503 discloses the study carried out on 10 cultivars of muscadine grapes that were separated into skin, seed, and pulp. Each fruit part and the leaves from the corresponding varieties were extracted for HPLC analysis of major phenolics. Gallic acid, (+)-catechin, and epicatechin were the major phenolics in seeds, with average values of 6.9, 558.4, and 1299.4 mg/100 g of fresh weight (FW), respectively. In the skins, ellagic acid, myricetin, quercetin, kaempferol, and transresveratrol were the major phenolics, with respective average values of 16.5, 8.4, 1.8, 0.6, and 0.1 mg/100 g of FW. Average total phenolics were 2178.8, 374.6, 23.8, and 351.6 mg/g gallic acid equivalent in seed, skin, pulp, and leaves, respectively. Antioxidant capacity values were, on average, 2.4, 12.8, 281.3, and 236.1 µM TEAC/g of FW for pulps, skins, seeds, and leaves, respectively.

Document SANDHU A K ET AL, "Effects of exogenous abscisic acid on antioxidant capacities, anthocyanins, and flavonol contents of muscadine grape (Vitis rotundifolia) skins", FOOD CHEMISTRY, ELSEVIER LTD, NL, vol. 126, no. 3, ISSN 0308-8146, pages 982 - 988, discloses the results of a study evaluating the effect of exogenous abscisic acid (ABA) on the antioxidant capacity and phenolic content of muscadine grape skins (cvs. Noble and Alachua). Freeze dried skin of grapes comprises (on fresh weight basis) 4,4 mg/g of total phenolics (Table 2, variety Noble), 3,46 mg/g ellagic acid, 0,36 mg/g myricetin and 0,27 mg/g quercetin.

US6238673 (HOWARD ALAN NORMAN) discloses a method of producing a polyphenol-containing composition derived from grapes, the method including: preparing a liquid grape extract which includes polyphenols; contacting the liquid extract with a separation medium which fractionates the components of the extract; and recovering that fraction in which the polyphenols are present. In particular, example 1 describes a barbera wine dealcoholized powder containing 0,7 mg/g myricetin, 4,3 mg/g quercetin, 13,9 mg/g catechin and 11,3 mg/g epicatechin. This document also discloses a method of enriching the composition with added flavonol. The flavonol-containing composition has antioxidative effects.

ES2217966 (JORDA QUILES LEONARDO) discloses a method of preparing a non-dairy food product enriched with antioxidants obtained by adding an extract from black grape seeds, skin and stalk to the food product to be enriched. The disclosed extract comprises 1,2 mg/g quercetin and 8,8 mg/g catechin.

US2010015316 (YING, W et al.) discloses a method for producing grape extract for use in dietary supplements. The grapes are processed to obtain a powdered or liquid extract concentrated in polyphenols which is added to dietary supplements.

Document RODRIGO, R et al. "Modulation of endogenous antioxidant system by wine polyphenols in human disease", Clinica Chimica Acta, 20.02.2011 Vol 412, n 5-6, pages 410-424 ISSN: 009-8981, discloses several grape extracts compositions containing different polyphenols.

WO 9739632 (HENKEL CORPORATION) discloses different natural antioxidant compositions comprising crushed and lyophilized grape seeds.

Document RODRIGUEZ MONTALEGRE, R. ET AL., "Phenolic compounds in skins and seeds of ten grape Vitis vinifera varieties grown in a warm climate.", JOURNAL OF FOOD COMPOSITION AND ANALYSIS, vol. 19, no. 6-7, pages 687 - 693, describes a study analyzing non-anthocyanin phenols present in the skins and seeds of 70 grape samples belonging to 10 cultivars. Grape skins contained tartaric esters of hydroxycinnamic acids (6-45 mg/kg of grape), monomeric and dimeric flavan-3-ols (9-96 mg/kg) and flavonols (25-197 mg/kg). The seed constituents comprised almost exclusively flavan-3-ols with concentration ranges of 330-1390 mg/kg.

ES 2319032 (MORO GONZALEZ CARLOS) discloses a process for extracting polyphenols from grape marc from distillation. The method described involves the following stages:
a) extraction of the pomace by continuous counter current diffusion using as an extractant a 50:50 solution of ethanol: acidified water at pH 1, at a temperature of 40-55° C and during 3-4 hours, with the pomace: extractant ratio in the interval being 1:2-3.5,to obtain an extraction pomace and an extraction juice;
b) pressing of the extraction pomace resulting from stage a) at a pressure between 2 and 10 kg/cm2, obtaining a press pomace and a press juice;
c) mixing of the extraction juice obtained at stage a) and the press juice obtained at stage b), followed by cooling of the resulting mix to 25°C, purification of the same by 100 micron filtering and centrifugation, obtaining a clarified juice and a solid residue;
d) stabilisation of the clarified juice obtained at stage c) by adding sodium alginate in a concentration of 0.2-0.6 g/L of clarified juice;
e) concentration of the stabilised juice obtained at stage d) in a vacuum evaporator at 60-70°C until the volume of stabilised juice is reduced to approximately 50% to obtain a polyphenolic extract with an antioxidant power in the range of 150,000 to 250,000 mg gallic acid/kg of dry pomace;

US 2004/166179 (ANZAGHI PIERGIORGIO ET AL.), teaches that liquid red wine vinasse was mixed with maltodextrin followed by freeze-drying to obtain a poder having a high antioxidant capacity. Maltodextrin was used in order to enhance the bioavailability of the antioxidant complexes in vinasse.

US 2011/177182 (IANIRO TEODORO T ET AL.), discloses a process for preparing an antioxidant capsule formulation, comprising mixing grape pomace with maltodextrin, followed by freeze-drying. However, maltodextrine is used in this process as a simple carrier, no specific purpose is disclosed.

### DESCRIPTION OF THE INVENTION

The primary object of this invention is an extract derived from grapes (*Vitis vinifera*), which has specific compositions heretofore unknown and beneficial properties for health, and is appropriate for human consumption (as well as for topical use, as will be seen further on). The composition of the grape extract includes one or more polyphenol compounds in a weight percentage of the total of the composition of the extract of between 5% and 70%, both limits included, which can be identified through high-performance liquid chromatography (HPLC) analysis. Said extract is in dry form.

In the present specification, it should be understood that said percentage (5% - 70% both limits included) refers collectively to all the polyphenols present in the composition of the extract, whether the extract contains just one polyphenol, or two or more polyphenols. Preferably, the polyphenol compound or compounds comprised in the grape extract are selected independently of one another from a group consisting of flavan-3-ol, myricetin, quercetin, ellagic acid, and any combination thereof. Even more preferably, the flavan-3-ol is selected from a group made up by: catechin, epicatechin, epigallocatechin, catechin gallate and any combination thereof.

It should be borne in mind that when a range of values is cited in the present specification, its lower and upper limits always fall within the scope of the invention, as specific embodiments of the same. The invention is as defined by the appended claims.

The dry grape extract as defined in claim 1 comprises the following concentration of polyphenols, by weight:
- ellagic acid: between 1 and 5 mg/g of the composition of the extract;
- myricetin: between 0.5 and 4 mg/g of the composition of the extract;
- quercetin: between 0.5 and 4 mg/g of the composition of the extract; and
- flavan-3-ols: between 1.1 and 10 mg/g of the composition of the extract.

The flavan-3-ols comprised by the extract are the following:
- catechin: between 0.5 and 5 mg/g of the composition of the extract;
- epicatechin: between 0.1 and 1 mg/g of the composition of the extract;
- epigallocatechin: between 0.5 and 4 mg/g of the composition of the extract; and
- catechin gallate: between 0.05 and 1 mg/g of the composition of the extract.

The described grape extract, in any of its variants, may be extracted directly from white and/or red grapes, or indirectly from vinification-process by-products. These by-products may be, for example, lees, marc, grape skins, etc., and any combination thereof, although the most preferable are grape skins, i.e. the skin around the grape pulp after pressing. What is especially preferable is that the grape extract come from red Tempranillo grapes from the Duero valley (Spain).

It is possible (optionally) for the extract to contain not just polyphenols, but also one or more other components and molecules derived from the source grapes or from by-products of the vinification process, for instance dietary fiber, vegetable protein, etc., and which differentiate said extract from a simple mixture of polyphenols produced artificially in a plant or laboratory. In a preferred instance, the extract object of the present invention may also contain the sum total of polyphenols present in the grape (among which phenolic acids -ellagic acid, gallic acid...-, stilbenes -resveratrol...-, and flavonoids - flavonols, flavan-3-ols...- are to be found), and/or the total of polyphenols present in the by-products of the vinification process.

The grape extract herein described is preferably obtained from a liquid composition extracted from grapes or from at least one by-product derived from a vinification process, which liquid composition undergoes a drying process. It is particularly preferable for said liquid composition, which contains the polyphenol or polyphenols, to be obtained according to the obtainment method described in application ES2319032. This method is based on collecting the marc left over after the vinification process, which then undergoes a continuous countercurrent diffusion solid-liquid extraction process.

The grape-derived liquid composition used to obtain the extract described in the present invention is preferably dried by freeze-drying and/or spray-drying.

In a preferred embodiment, the dry extract additionally comprises an excipient, a diluent, or a vehicle, for instance maltodextrine, silicon dioxide, xanthan gum, etc., used in the process of drying the liquid composition through freeze-drying and/or spray-drying.

In light of the above, the present invention likewise covers a process for obtaining the grape extract herein described, which comprises drying a liquid composition extracted from grapes or from at least one by-product derived from a vinification process that comprises one or more polyphenol compounds, till obtaining a dry product comprising at least one of the polyphenol compounds in a weight percentage of the total of between 5% and 70%, both limits included, said percentage relating to the total polyphenols present in the composition of the extract, whether this be one, two or more. As stated previously, said drying process preferably consists of freeze-drying and/or spray-drying the liquid composition. Even more preferably, said drying employs an excipient, a diluent, or a vehicle, for instance maltodextrine, silicon dioxide, xanthan gum, etc.

A further object of this invention is a functional dietary (nutritional) supplement characterized in that it comprises the grape extract described above as a functional ingredient. The grape extract acts directly as a functional dietary supplement.

Said dietary supplement is characterized in that it comprises at least between 10 mg and 1 g of the grape extract. Preferably, this dietary supplement is characterized in that it comprises, in addition to the grape extract as a functional ingredient, at least one physiologically acceptable substance having properties selected from the group made up by: nutritional, antioxidant, therapeutic, flavoring, aromatic, coloring or other such properties.

It is likewise preferable for the edible dietary composition to additionally comprise at least one substance selected from the group made up by: Vitamins (Vitamin A, Vitamin B1, Vitamin B2, Vitamin B3, Vitamin B6, Vitamin B9, Vitamin B12, Vitamin C, Vitamin E), Trace Dietary Elements (for example, Zinc, Copper, Magnesium, Selenium, Manganese), phytochemicals (for example Carotenoids, Lutein, Zeaxanthin, Astaxanthin), Collagen, Type-II Collagen, Chondroitin Sulphate, Hyaluronic Acid, Omega-3 Fatty Acids, Hydroxytol, Glutathione, Glucosamine, phyto extracts enriched with proanthocyanidins and phyto extracts from other plant products (such as, optionally, pomegranate, green tea, olive, *Polygonum cuspidatum).*

Both the dry grape extract described in this specification, and the dietary supplement that comprises it (as a functional ingredient) may be directly eaten by human beings. As such, the purview of the present invention includes not only the use of the grape extract as a dietary supplement, but also the use of the grape extract as a functional ingredient in food or in a dietary composition.

In this way, both the grape extract and the nutritional or dietary supplement described above may be packaged in bulk or in single doses. In general, the extract and the dietary supplement may be packaged in cans, drums, cartons or any other type of packaging known in the field. When packaged in single doses, said extract and said composition which comprise it may take the form of a tablet, a capsule or a pill.

It has been shown that the effect of the grape extract herein described, or of a dietary supplement comprising said grape extract, offers beneficial properties for human health, fighting oxidative stress and improving the parameters associated with cardiovascular disease. For example, it has been seen how one benefit derived from the consumption of this extract, or the dietary supplement that contains it, is reduced levels of blood lipoprotein-cholesterol and total cholesterol levels, and an increase in the antioxidant capacity of cellular plasma and vitamin E concentration, among other physiological parameters.

The present disclosure further includes a foodstuff characterized in that its composition comprises as a functional ingredient the described grape extract or the nutritional supplement which can be obtained thereof, mixed together with at least one dietary ingredient. In a preferred embodiment of the foodstuff, it comprises as a dietary ingredient one of the products selected from the group made up by meat products, dairy products, milk derivatives, sauces, food paste, bakery and pastry products, and the like, and any combination thereof.

In another preferred embodiment, the foodstuff is a beverage, and the grape extract or the nutritional supplement that comprises it is mixed with a physiologically acceptable liquid.

These foods and beverages achieve the same antioxidant results in health as the grape extract that they comprise.

The use of the described grape extract, in any of its variants, for the production of different functional products is also disclosed. These products include foodstuffs and dietary supplements for human consumption. Said composition may be used to reduce the levels of blood lipoprotein-cholesterol and total cholesterol, and/or increase the antioxidant capacity of plasma and vitamin E concentration. In this way, the grape extract of the present invention can be used to prevent cardiovascular diseases and conditions caused by oxidative stress as a result of metabolism.

Also described are topical use of the described grape extract. Claimed is its use in the production of cosmetic compositions such as moisturizers, clansers, ect.

### Comparative examples

### Example 1. Deriving the polyphenol grape extract from red wine

Red Tempranillo grapes from the Duero Valley were used as a raw material for the extract. Specifically, grape skins were used, i.e. the skin around the grape pulp after pressing.

The method used to obtain a liquid composition to be used as a raw material for the dry grape extract is the one described in the application ES2319032. This method is based on collecting the marc left over after the vinification process, which then undergoes a continuous countercurrent diffusion solid-liquid extraction process.

The obtained polyphenol extract is characterized in that it comprises not only polyphenols, but also other components and molecules from the source grapes, which characterize said liquid composition.

Afterwards the liquid composition from the grapes underwent a spray-drying and/or freeze-drying process, to obtain the dry grape extract of the present invention.

### Example 2. Format and composition of the dry grape extract obtained in Example 1 in capsules

The dry extract was put into hard gelatin capsules in such a way that the capsules contained 374.5 milligrams, distributed as follows:
- dry extract: 350 mg.
- Microcrystalline cellulose: 17.15 mg.
- Magnesium stearate: 7.35 mg.

### Example 3. Characterization of the dry red-grape extract used to prepare the capsules in Example 2.

The polyphenol composition of the dry red-grape extract is listed below:

| | |
|---|---|
| - ellagic acid: | 1-5 mg/g |
| - myricetin: | 0.5-4 mg/g |
| - quercetin: | 0.5-4 mg/g |
| - flavan-3-ols: | 1.1-10 mg/g |
| ∘ catechin: | 0.5-5 mg/g |
| ∘ epicatechin: | 0.1-1 mg/g |
| ∘ epigallocatechin: | 0.5-4 mg/g |
| ∘ catechin gallate: | 0.05-1 mg/g |

The polyphenol composition of the extract is characterized by its high concentration of ellagic acid, as the extract is completely grape-based.

### Example 4. Trial with volunteers using the grape extract to determine its healthful properties in healthy volunteers.

A parallel, placebo-controlled, double-blind, randomized, exploratory, clinical nutritional trial was conducted with a sample of healthy volunteers using the composition cited in Example 2, for the purpose of assessing its healthful properties and the tolerability of the extract over different parameters indicative of cardiovascular risk and oxidative stress.

Each participant in the trial took 700 mg/day of the grape extract, object of the present invention, or a placebo, for 8 weeks (56 days). Between one and four weeks prior to the beginning of the experimental phase, the potential participants were selected. Said selection process was carried out based on whether or not they met the inclusion/exclusion criteria for the trial, by means of: medical history (anamnesis and physical examination), laboratory tests (hemogram, biochemistry, systematic and sediment urine analysis), and complementary tests (ECG).

During the experimental phase three visits were made to the trial center: at the beginning (baseline visit, V0), halfway through (halfway visit, V28) and at the end of the study (end visit, V56). Following the experimental period, the volunteers underwent another checkup involving: physical examination, laboratory tests (hemogram, biochemistry, systematic and sediment urine analysis) and complementary tests (ECG).

### Selecting the volunteers.

The volunteers who participated in the trial met the following inclusion criteria:
- Subjects from both sexes between 18 and 65 years of age, both inclusive.
- Medical history, review of systems and laboratory tests within normal parameters (particularly in terms of the gastrointestinal tract), without evidence of significant, organic or psychiatric disease.
- Sufficient cultural level and understanding of the clinical trial.
- BMI between 19 and 30 Kg/m2.
- Agreement to voluntarily participate in the trial and written informed consent.
- Having LDL-cholesterol levels between 130 and 190 mg/dl. In the case of those having two or more cardiovascular risk factors, have LDL-cholesterol levels between 100 and 190 mg/dl.
The exclusion criteria were as follows:
- Having LDL-cholesterol levels above 190 mg/dl.
- Having triglyceride levels above 350 mg/dl.
- Having suffered any kind of ischemic cardiovascular event within the last 6 months.
- Undergoing or having undergone treatment with hypolipidemic drugs or any other type of treatment for hypercholesterolemia during the 4 weeks prior to the beginning of the study.
- Pregnant or nursing women.
- Being hypersensitive to any component of the product in the trial or the placebo.
- Having participated in another clinical trial during the three months prior to the beginning of the trial in question.

### Experimental design of the trial.

Each subject took two capsules per day for 56 days (8 weeks), before breakfast with water or juice. The participants were divided into two parallel trial groups, one of which received the extract while the other received the placebo.

Grape extract: capsules containing the extract with the composition specifications described in Example 2. Taken orally.

Control extract: placebo, composition: microcrystalline cellulose (315 mg) and magnesium stearate (6 mg). Taken orally.

All participants in the trial followed a series of recommendations concerning hygiene and diet, which were provided to them at the beginning of the trial by the head researcher:
- not to start or alter any hormone treatment during the trial unless duly justified.
- not to significantly change habits in the consumption of coffee or alcohol.
- not to undergo any treatment that might affect the parameters of the trial (weight, fat accumulation, feeling hungry/full...)

### Development of the trial.

Each volunteer made 3 visits to the trial center during the experimental phase: at the beginning (visit 0); halfway through (visit 28); and at the end of the study (visit 56).

During each of the visits, various data were collected in relation to the parameters of the trial: measurement of weight; anthropometric measurements (waist and hip circumference); impedance testing, and a blood and urine test, in which various biochemical indicators were analyzed such as, lipid profile, fasting blood glucose, and various oxidative stress markers such as plasma antioxidant capacity, through ORAC, levels of vitamins C and E, lipid peroxidation (TBARS) and F2-isoprostanes in urine, oxidized LDL and Tumor Necrosis Factor alpha (TNF-α).

Following the experimental period, the participants underwent a checkup involving: physical examination, laboratory tests (hemogram, biochemistry, systematic and sediment urine analysis) and complementary tests (ECG).

The assessment of the effect of consuming the capsules with grape extract on the parameters of the trial was carried out by evaluating the differences between the initial and end values of the target parameters specified above.

The main assessment variable was the evolution in LDL-cholesterol by the end of the extract consumption period.

Other variables were also evaluated, such as total cholesterol, HDL-cholesterol, LDL/HDL ratio, triglycerides, fasting blood glucose, weight, anthropometric measurements (waist circumference and hip circumference), percentage of body fat; and various parameters related to oxidative stress and inflammation, such as plasma antioxidant capacity, lipid peroxidation, F2-isoprostanes in urine, levels of vitamins C and E, ox-LDL and TNF-α, following completion of the treatment.

The variables of the trial, then, were as follows:
Main variables:
   - LDL-cholesterol.
Secondary variables:
   - Total cholesterol.
   - HDL-chol.
   - LDL/HDL.
   - Triglycerides.
   - Fasting blood glucose.
   - Weight.
   - Waist circumference and hip circumference.
   - % of body fat.
   - Plasma antioxidant capacity (ORAC).
   - Lipid peroxidation (TBARS).
   - F2-isoprostanes.
   - Vitamin C and E levels.
   - Oxidized LDL.
   - Tumor necrosis factor (TNF-alfa).

### Results

Statistically significant differences were observed between the extract and the placebo extract in several of the trial variables. Namely, there was an improvement in parameters associated with cardiovascular risk, for instance LDL-cholesterol levels and total cholesterol, along with an increase in plasma antioxidant capacity (ORAC) and in vitamin E levels.

In terms of analysis by individual treatments:
- The grape extract brought about a statistically significant decrease in LDL-cholesterol and total cholesterol levels, which was not observed in the placebo group.
- Consumption of the grape extract brought about a statistically significant increase in plasma antioxidant capacity (ORAC), which was not observed in the placebo group, as indicated in Table 1.

**Table 1. Evolution of the variable ORAC for each of the two experimental groups, by visit.**

| TREATMENT GROUP | VALUE | INITIAL ORAC | HALFWAY ORAC | END ORAC |
|---|---|---|---|---|
| GROUP A (EXTRACT) | N | 30 | 30 | 30 |
| | Mean | 51.23 | 61.10 | 65.63 |
| | Std. Dev. | 4.80 | 5.05 | 5.79 |
| | Median | 50.00 | 61.00 | 65.00 |
| GROUP B (PLACEBO) | N | 30 | 30 | 30 |
| | Mean | 56.70 | 57.93 | 57.80 |
| | Std. Dev. | 7.71 | 7.74 | 7.71 |
| | Median | 58.00 | 58.50 | 57.50 |

In terms of product safety, there were no differences detected, between the two extract intakes, in transaminase or creatinine levels.

In light of all the above, the grape extract did indeed prove to carry out antioxidant activity and improve the parameters related to cardiovascular risk. The healthful properties of the grape extract were thus demonstrated, as well as its high tolerance and safety.

The examples provided show the optimal characteristics of the grape extract herein described and the dietary supplements that comprise it, as well as its beneficial properties, of which its antioxidant capacity and its beneficial cardiovascular effects on human health are worth mentioning.

### WORKS CITED

Aldini G, et al. 2003. Life Sci.; 73: 2883-2898.
Burns J, et al. 2000. J. Agric. Food Chem.; 48: 220-230
Chance B, et al. 1979. Physiol. Rev.; 59: 527-605
D'Archivio M, et al. 2007. Ann. Ist. Super Sanitá; 43(4): 348-361
Elejalde JI. 2001. An. Med. Interna (Madrid); 18 (6): 326-335
Gerschman R. 1954. Science; 119: 623-626
Harman D. 1993. Drugs and ageing; 3: 60-80
Hazen SL. 2000. Free Radic. Biol. Med.; 28: 1683-1684
Higdon JV & Frei B. 2003. Crit. Rev. Food Sci. Nutr.; 43: 89-143
Klebanoff SJ. 1980. Ann. Intern. Med.; 93: 480-487
Kuulasmaa K, et al. 2000. Lancet; 355: 675-687
Nigdikar SV, et al. 1998. Am. J. Clin. Nutr.; 68: 258-265
Oberley TD & Oberley LW. 1997. Histopatol.; 12(2); 525-535
Sies H. 1986. Angew Chem.; 25: 1058-71
Stoclet JC, et al. 2004. Eur. J. Pharmacol.; 500: 299-313
Turrens J. 1994. Antioxidantes y Calidad de Vida; 1: 16-19
Vieira O, et al. 1998. Br. J. Pharmacol.; 123: 565-573

## Claims

1. Dry grape extract obtainable by method of claim 6 **characterized in that** it comprises a weight percentage of polyphenol compounds with respect to the total of the composition of the extract of between 5% and 70%, both limits included, wherein the concentration by weight of said polyphenol compounds is as follows
- ellagic acid: between 1 and 5 mg/g of the composition of the extract;
- myricetin: between 0.5 and 4 mg/g of the composition of the extract;
- quercetin: between 0.5 and 4 mg/g of the composition of the extract; and
- flavan-3-ols: between 1.1 and 10 mg/g of the composition of the extract, wherein said flavan-3-ols are:
• catechin: between 0.5 and 5 mg/g of the composition of the extract;
• epicatechin: between 0.1 and 1 mg/g of the composition of the extract;
• epigallocatechin: between 0.5 and 4 mg/g of the composition of the extract; and
• catechin gallate: between 0.05 and 1 mg/g of the composition of the extract.

2. Extract according to claim 1, derived from white grapes, from red grapes, from a mixture of white grapes and red grapes, or from by-products of a vinification process.

3. Extract according to the preceding claim, wherein the by-products are selected from among the group made up by lees, marc, grape skins and any combination thereof.

4. Extract according to any one of the preceding claims, which comprises at least one second component derived from grapes or from the by-products of a vinification process.

5. Extract according to any one of the preceding claims, which comprises the total polyphenol compounds found in the grape.

6. Method for obtaining the dry grape extract described in any one of the claims 1 to 5, which, on the basis of distilled grape pomaces, involves the following stages:
a) extraction of the pomace by continuous counter current diffusion using as an extractant a 50:50 solution of ethanol: acidified water at pH 1, at a temperature of 40-55° C and during 3-4 hours, with the pomace: extractant ratio in the interval being 1:2-3.5,to obtain an extraction pomace and an extraction juice;
b) pressing of the extraction pomace resulting from stage a) at a pressure between 2 and 10 kg/cm2, obtaining a press pomace and a press juice;
c) mixing of the extraction juice obtained at stage a) and the press juice obtained at stage b), followed by cooling of the resulting mix to 25°C, purification of the same by 100 micron filtering and centrifugation, obtaining a clarified juice and a solid residue;
d) stabilisation of the clarified juice obtained at stage c) by adding sodium alginate in a concentration of 0.2-0.6 g/L of clarified juice;
e) concentration of the stabilised juice obtained at stage d) in a vacuum evaporator at 60-70°C until the volume of stabilised juice is reduced to approximately 50% to obtain a polyphenolic extract with an antioxidant power in the range of 150,000 to 250,000 mg gallic acid/kg of dry pomace;
**characterized in that** it comprises drying the liquid composition obtained at the previous stages that comprises flavan-3-ol, myricetin, quercetin and ellagic acid as polyphenol compounds, till obtaining a dry product comprising said extract, where the percentage by weight of the total polyphenolic compounds present in the extract is between 5% and 70%, both inclusive, with respect to the total weight of the extract composition, wherein the drying is carried out by freeze-drying and/or spray-drying,
and wherein maltodextrine, silicon dioxide or xanthan gum is employed in the drying process as an excipient, a diluent, or a vehicle.

7. Dietary supplement that comprises the grape extract described in any one of the claims 1 to 5 as a functional ingredient, **characterized in that** it comprises between 10 mg and 1 g of the grape extract.

8. Dietary supplement according to claim 7, which further comprises at least one physiologically acceptable substance having properties selected from the group made up by: nutritional, antioxidant, therapeutic, flavoring, aromatic, coloring or other such properties.

9. Dietary supplement according to any one of the claims 7 or 8, which further comprises at least one substance selected from among the group made up by: Vitamins, Trace Dietary Elements, Phytochemicals, Collagen, Type II Collagen, Chondroitin Sulphate, Hyaluronic Acid, Omega-3 Fatty Acids, Hydroxytol, Glutathione, Glucosamine, phyto extracts enriched with proanthocyanidins and phyto extracts from other plant products.

10. Dietary supplement according to any one of the claims 7 to 9, which is packaged in bulk or in single doses.

11. Dietary supplement according to the preceding claim, which when packaged in single doses takes the form of a tablet, a capsule or a pill.

12. Dietary supplement according to any one of the claims 7 to 9, **characterized in that** it is mixed together with at least one dietary ingredient for the production of a foodstuff.

13. Dietary supplement according to the preceding claim, wherein the dietary ingredient is one of the products selected from among the group made up by meat products, dairy products, milk derivatives, sauces, food paste, bakery and pastry products, and any combination thereof.

14. Dietary supplement according to claim 13, **characterized in that** the dietary supplement it is mixed with a physiologically acceptable liquid for the production of a beverage.

15. Grape extract according to any one of the claims 1 to 5 for use in reducing the levels of blood lipoprotein-cholesterol and total cholesterol, and/or for use in increasing plasma antioxidant capacity and vitamin E concentration.

16. Use of the grape extract described in any one of the claims 1 to 5 for the production of cosmetic products.

## Patentansprüche

1. Trockener Traubenextrakt, erhältlich durch das Verfahren gemäß Anspruch 6, **dadurch gekennzeichnet, dass** er einen Gewichtsanteil an Polyphenolverbindungen bezogen auf die Gesamtzusammensetzung des Extrakts von zwischen 5% und 70%, beide Grenzwerte eingeschlossen, umfasst, wobei die Gewichtskonzentration der Polyphenolverbindungen ist wie folgt:
- Ellaginsäure: zwischen 1 und 5 mg/g der Zusammensetzung des Extrakts;
- Myricetin: zwischen 0,5 und 4 mg/g der Zusammensetzung des Extrakts;
- Quercetin: zwischen 0,5 und 4 mg/g der Zusammensetzung des Extrakts; und
- Flavan-3-ole: zwischen 1,1 und 10 mg/g der Zusammensetzung des Extrakts, wobei die Flavan-3-ole sind:
• Catechin: zwischen 0,5 und 5 mg/g der Zusammensetzung des Extrakts;
• Epicatechin: zwischen 0,1 und 1 mg/g der Zusammensetzung des Extrakts;
• Epigallocatechin: zwischen 0,5 und 4 mg/g der Zusammensetzung des Extrakts; und
• Catechingallat: zwischen 0,05 und 1 mg/g der Zusammensetzung des Extrakts.

2. Extrakt gemäß Anspruch 1, abgeleitet von weißen Trauben, von roten Trauben, von einem Gemisch von weißen Trauben und roten Trauben oder von Nebenprodukten eines Weinherstellungsverfahrens.

3. Extrakt gemäß dem vorstehenden Anspruch, wobei die Nebenprodukte ausgewählt sind aus der Gruppe bestehend aus Trub, Trester, Traubenschalen und einer beliebigen Kombination davon.

4. Extrakt gemäß einem der vorstehenden Ansprüche, umfassend wenigstens eine zweite Komponente abgeleitet von Trauben oder von den Nebenprodukten eines Weinherstellungsverfahrens.

5. Extrakt gemäß einem der vorstehenden Ansprüche, umfassend die gesamten in der Traube gefundenen Polyphenolverbindungen.

6. Verfahren zum Erhalten des trockenen Traubenextrakts gemäß einem der Ansprüche 1 bis 5, das auf der Basis von destilliertem Traubentrester folgende Schritte umfasst:
a) Extraktion des Tresters durch kontinuierliche Gegenstromdiffusion unter Verwendung einer 50:50-Lösung von Ethanol:gesäuertes Wasser mit pH 1 mit einer Temperatur von 40-55°C als Extraktionsmittel über einen Zeitraum von 3-4 Stunden, wobei das Trester:Extraktionsmittel-Verhältnis in dem Bereich von 1:2-3,5 liegt, um einen Extraktionstrester und einen Extraktionssaft zu erhalten;
b) Pressen des bei Schritt a) erhaltenen Extraktionstresters mit einem Druck von zwischen 2 und 10 kg/cm², um einen Presstrester und einen Presssaft zu erhalten;
c) Mischen des bei Schritt a) erhaltenen Extraktionssafts und des bei Schritt b) erhaltenen Presssafts, gefolgt von Kühlen des erhaltenen Gemischs auf 25°C, Reinigen davon durch 100-Mikrometer-Filtration und Zentrifugation, Erhalten eines geklärten Safts und eines festen Rückstands;
d) Stabilisieren des bei Schritt c) erhaltenen geklärten Safts durch Zugeben von Natriumalginat mit einer Konzentration von 0,2-0,6 g/l des geklärten Safts;
e) Konzentrieren des bei Schritt d) erhaltenen stabilisierten Safts in einem Unterdruckverdampfer bei 60-70°C, bis das Volumen des stabilisierten Safts auf etwa 50% verringert ist, um einen Polyphenolextrakt mit einer Antioxidationskraft in dem Bereich von 150.000 bis 250.000 mg Gallensäure/kg trockenem Trester zu erhalten;
**dadurch gekennzeichnet, dass** es Trocknen der bei den vorangehenden Schritten erhaltenen flüssigen Zusammensetzung umfasst, die Flavan-3-ol, Myricetin, Quercetin und Ellaginsäure als Polyphenolverbindungen umfasst, bis ein trockenes Produkt erhalten ist, das den Extrakt umfasst, wobei der Gewichtsanteil der gesamten Polyphenolverbindungen, die in dem Extrakt vorhanden sind, zwischen jeweils einschließlich 5% und 70% bezogen auf das Gesamtgewicht der Extraktzusammensetzung beträgt,
wobei das Trocknen durch Gefriertrocknen und/oder Sprühtrocknen durchgeführt wird,
und wobei bei dem Trocknungsverfahren Maltodextrin, Siliciumdioxid oder Xanthangummi als Hilfsstoff, Verdünnungsmittel oder Träger eingesetzt wird.

7. Nahrungsergänzungsmittel, umfassend den Traubenextrakt gemäß einem der Ansprüche 1 bis 5 als funktionellen Bestandteil, **dadurch gekennzeichnet, dass** es zwischen 10 mg und 1 g des Traubenextrakts umfasst.

8. Nahrungsergänzungsmittel gemäß Anspruch 7, ferner umfassend wenigstens einen physiologisch annehmbaren Stoff mit Eigenschaften ausgewählt aus der Gruppe bestehend aus: Ernährungs-, Antioxidations-, therapeutischen, aromatisierenden, Aroma-, Farb- und anderen derartigen Eigenschaften.

9. Nahrungsergänzungsmittel gemäß einem der Ansprüche 7 oder 8, ferner umfassend wenigstens einen Stoff ausgewählt aus der Gruppe bestehend aus: Vitaminen, Nahrungs-Spurenelementen, phytochemischen Stoffen, Collagen, Typ-II-Collagen, Chondroitinsulfat, Hyaluronsäure, Omega-3-Fettsäuren, Hydroxytol, Gutathion, Glucosamin, Phytoextrakten, die mit Proanthocyanidinen angereichert sind, und Phytoextrakten von anderen Pflanzenprodukten.

10. Nahrungsergänzungsmittel gemäß einem der Ansprüche 7 bis 9, das in Masse oder in Einzeldosen verpackt ist.

11. Nahrungsergänzungsmittel gemäß dem vorstehenden Anspruch, das, wenn in Einzeldosen verpackt, die Form einer Tablette, einer Kapsel oder einer Pille annimmt.

12. Nahrungsergänzungsmittel gemäß einem der Ansprüche 7 bis 9, **dadurch gekennzeichnet, dass** es mit wenigstens einem Nahrungsbestandteil für die Herstellung eines Lebensmittels zusammengemischt ist.

13. Nahrungsergänzungsmittel gemäß dem vorstehenden Anspruch, wobei der Nahrungsbestandteil eines der Produkte ausgewählt aus der Gruppe bestehend aus Fleischprodukten, Milchprodukten, Milchderivaten, Saucen, Lebensmittelpaste, Backwaren und Gebäck und einer beliebigen Kombination davon ist.

14. Nahrungsergänzungsmittel gemäß Anspruch 13, **dadurch gekennzeichnet, dass** das Nahrungsergänzungsmittel mit einer physiologisch annehmbaren Flüssigkeit für die Herstellung eines Getränks gemischt ist.

15. Traubenextrakt gemäß einem der Ansprüche 1 bis 5 für die Verwendung zum Verringern der Spiegel von Blut-Lipoprotein-Cholesterol und Gesamtcholesterol und/oder zur Verwendung zum Erhöhen des Plasma-Antioxidationsvermögens und der Vitamin-E-Konzentration.

16. Verwendung des Traubenextrakts gemäß einem der Ansprüche 1 bis 5 zur Herstellung kosmetischer Produkte.

## Revendications

1. Extrait sec de raisin pouvant être obtenu par une méthode selon la revendication 6, **caractérisé en ce qu'**il comprend un pourcentage pondéral de composés de polyphénol par rapport au total de la composition de l'extrait compris entre 5% et 70%, les deux limites étant incluses, où la concentration en poids desdits composés de polyphénol est comme suit:
- acide ellagique: entre 1 et 5 mg/g de la composition de l'extrait;
- myricétine: entre 0,5 et 4 mg/g de la composition de l'extrait;
- quercétine: entre 0,5 et 4 mg/g de la composition de l'extrait; et
- flavan-3-ols: entre 1,1 et 10 mg/g de la composition de l'extrait; où lesdits flavan-3-ols sont:
• catéchine: entre 0,5 et 5 mg/g de la composition de l'extrait;
• épicatéchine: entre 0,1 et 1 mg/g de la composition de l'extrait;
• épigallocatéchine: entre 0,5 et 4 mg/g de la composition de l'extrait; et
• gallate de catéchine: entre 0,05 et 1 mg/g de la composition de l'extrait.

2. Extrait selon la revendication 1, dérivé de raisins blancs, de raisins rouges, d'un mélange de raisins blancs et de raisins rouges, ou de sous-produits d'un procédé de vinification.

3. Extrait selon la revendication précédente, dans lequel les sous-produits sont choisis dans le groupe constitué par les lies, le marc, les peaux de raisin, et une combinaison quelconque de ceux-ci.

4. Extrait selon l'une quelconque des revendications précédentes, comprenant au moins un deuxième composant dérivé de raisins ou de sous-produits d'un procédé de vinification.

5. Extrait selon l'une quelconque des revendications précédentes, comprenant les composés de polyphénol totaux rencontrés dans les raisins.

6. Méthode d'obtention de l'extrait sec de raisin décrit selon l'une quelconque des revendications 1 à 5, qui, sur la base de marc de raisin distillé, met en jeu les étapes suivantes:
a) l'extraction du marc par une diffusion en contre-courant continue en utilisant comme agent d'extraction une solution 50:50 d'éthanol:eau acidifiée à pH 1, à une température de 40-55°C et pendant 3-4 heures, le rapport marc:agent d'extraction se trouvant dans l'intervalle de 1:2-3,5, afin d'obtenir un marc d'extraction et un jus d'extraction ;
b)le pressage du marc d'extraction résultant de l'étape a) à une pression comprise entre 2 et 10 kg/cm², pour obtenir un marc de presse et un jus de presse ;
c) le mélange du jus d'extraction obtenu dans l'étape a) et du jus de presse obtenu dans l'étape b), suivi du refroidissement du mélange résultant jusqu'à 25°C, la purification de celui-ci par une filtration sur 100 µm et une centrifugation, pour obtenir un jus clarifié et un résidu solide ;
d)la stabilisation du jus clarifié obtenu dans l'étape c) par l'addition d'alginate de sodium selon une concentration de 0,2-0,6 g/l de jus clarifié ;
e)la concentration du jus stabilisé obtenu dans l'étape d) dans un évaporateur sous vide à 60-70°C, jusqu'à ce que le volume de jus stabilisé soit réduit jusqu'à environ 50% afin d'obtenir un extrait polyphénolique ayant un pouvoir antioxydant dans la plage allant de 150.000 à 250.000 mg d'acide gallique/kg de marc sec ;
**caractérisée en ce qu'**elle comprend le séchage de la composition liquide obtenue dans les étapes précédentes qui comprend du flavan-3-ol, de la myricétine, de la quercétine et de l'acide ellagique comme composés de polyphénol, jusqu'à l'obtention d'un produit sec comprenant ledit extrait, où le pourcentage pondéral des composés phénoliques totaux présents dans l'extrait est compris entre 5% et 70%, les deux limites étant incluses, par rapport au poids total de la composition d'extrait,
où le séchage est effectué par lyophilisation et/ou séchage par pulvérisation,
et où des maltodextrines, du dioxyde de silicium ou de la gomme de xanthane sont employés dans le procédé de séchage comme excipient, diluant ou véhicule.

7. Complément alimentaire comprenant l'extrait de raisin décrit selon l'une quelconque des revendications 1 à 5 comme ingrédient fonctionnel, **caractérisé en ce qu'**il comprend entre 10 mg et 1 g de l'extrait de raisin.

8. Complément alimentaire selon la revendication 7, comprenant en outre au moins une substance physiologiquement acceptable ayant des propriétés choisies dans le groupe constitué par les propriétés nutritionnelles, antioxydantes, thérapeutiques, aromatisantes, aromatiques, colorantes ou de telles autres propriétés.

9. Complément alimentaire selon l'une quelconque des revendications 7 ou 8, comprenant en outre au moins une substance choisie dans le groupe constitué par les vitamines, les oligo-éléments alimentaires, les substances phytochimiques, le collagène, le collagène de type II, le sulfate de chondroïtine, l'acide hyaluronique, les acides gras oméga-3, l'hydroxytol, le glutathion, la glucosamine, les extraits végétaux enrichis en proanthocyanidines et les extraits végétaux issus d'autres produits végétaux.

10. Complément alimentaire selon l'une quelconque des revendications 7 à 9, qui est conditionné en vrac ou en doses unitaires.

11. Complément alimentaire selon la revendication précédente, qui, lors de son conditionnement en doses unitaires, prend la forme d'un comprimé, d'une capsule ou d'une pilule.

12. Complément alimentaire selon l'une quelconque des revendications 7 à 9, **caractérisé en ce qu'**il est mélangé conjointement avec au moins un ingrédient alimentaire pour la production d'un produit alimentaire.

13. Complément alimentaire selon la revendication précédente, dans lequel l'ingrédient alimentaire est l'un des produits choisis dans le groupe constitué par les produits carnés, les produits laitiers, les dérivés de lait, les sauces, les pâtes alimentaires, les produits de boulangerie et de pâtisserie, et une combinaison quelconque de ceux-ci.

14. Complément alimentaire selon la revendication 13, **caractérisé en ce que** le complément alimentaire est mélangé avec un liquide physiologiquement acceptable pour la production d'une boisson.

15. Extrait de raisin selon l'une quelconque des revendications 1 à 5, pour une utilisation dans la réduction des taux de lipoprotéines-cholestérol et de cholestérol total du sang, et/ou pour une utilisation dans l'augmentation de la capacité antioxydante du plasma et de la concentration en vitamine E.

16. Utilisation de l'extrait de raisin décrit selon l'une quelconque des revendications 1 à 5, pour la production de produits cosmétiques.
